Europäisches Patentamt

European Patent Office

Office européen des brevets

(19)

(11) Numéro de publication: **0 320 351**
**A1**

(12) **DEMANDE DE BREVET EUROPEEN**

(21) Numéro de dépôt: **88403057.8**

(22) Date de dépôt: **02.12.88**

(51) Int. Cl.⁴: **A 61 N 1/365**
**G 01 K 7/18**

(30) Priorité: **07.12.87 FR 8716992**

(43) Date de publication de la demande:
**14.06.89 Bulletin 89/24**

(84) Etats contractants désignés:
**AT BE CH DE ES FR GB GR IT LI LU NL SE**

(71) Demandeur: **BIOVALLEES Société Anonyme:**
**F-43150 Monastier sur Gazeille (FR)**

(72) Inventeur: **Brehier, Jacques**
**26, rue Barbier**
**F-72000 Le Mans (FR)**

(74) Mandataire: **Derambure, Christian et al**
**BUGNION ASSOCIES 55, rue Boissonade**
**F-75014 Paris (FR)**

(54) **Stimulateur cardiaque et procédé de réglage de ce stimulateur.**

(57) Stimulateur cardiaque du type piloté par la température veineuse du patient et comportant un boitier contenant une pile électrique, des moyens de stimulation associés à la pile, un connecteur (4) associé aux moyens de stimulation et une sonde destinée à être associée par une première partie extrême au connecteur (4) et comportant, à sa seconde partie extrême, d'une part un capteur de température, et d'autre part au moins un contact de stimulation relié électriquement au connecteur (4) par des conducteurs, et par le fait qu'il comporte, associée au boîtier et interposée en série entre l'un au moins des conducteurs de la sonde et les moyens de stimulation, une résistance électrique (12), réglable depuis l'extérieur du boîtier, ayant pour fonction de permettre de régler le stimulateur après mise en place quelle que soit la température veineuse et la résistance propre de la sonde.

FIG. 4

EP 0 320 351 A1

**Description**

## STIMULATEUR CARDIAQUE ET PROCEDE DE REGLAGE DE CE STIMULATEUR

L'invention concerne un stimulateur cardiaque implantable et un procédé de réglage de ce stimulateur.

Plus précisément, l'invention concerne un stimulateur cardiaque du type connu en soi piloté par la température veineuse du patient appareillé avec ce stimulateur, le stimulateur comportant en premier lieu un boitier contenant un pile électrique, des moyens de stimulation associés à la pile et un connecteur associé aux moyens de stimulation, et en second lieu une sonde destinée à être associée par une première partie extrême au connecteur et comportant, à sa seconde partie extrême, d'une part un capteur de température et, d'autre part, au moins un contact de stimulation, reliés électriquement au connecteur par des conducteurs (document EP 0096464 et WO 85 05279).

Des stimulateurs cardiaques de ce type présentent deux problèmes: le premier est la réalisation du capteur de température afin de répondre aux exigences de précision, de facilité de montage et de mise en place, de coût, de fiabilité, d'étanchéité requises pour ce type de capteur. Le second est le réglage du stimulateur. En effect, le capteur de température comporte une thermistance dont la résistance varie en fonction de la température. Or, cette résistance peut être du même ordre de grandeur que la résistance des conducteurs de la sonde où à tout le moins les résistances des conducteurs de la sonde sont importantes et non négligeables par rapport à la résistance de la thermistance du capteur de température. Si l'on souhaite utiliser des sondes du commerce dont la variation de résistance, toutes choses égales par ailleurs, est non négligeable par rapport à la sensibilité de la thermistance, on est obligé de procéder à un réglage du stimulateur cardiaque. On connait déjà un système de réglage électronique qui a cependant l'inconvénient d'être consommateur de courant, ce que l'on cherche à éviter, dans le cas d'un stimulateur cardiaque implanté. Une autre possibilité est d'utiliser des sondes calibrées quant à leur résistance mais cette solution a comme inconvénient, non seulement le fait de limiter l'emploi de sondes diverses, mais encore ne résout pas totalement le problème puisque la résistance peut varier du fait du montage même du stimulateur cardiaque notamment de la connection entre la sonde proprement dite et le connecteur du boitier, mais encore que la résistance de la sonde avec le capteur de température dépend de la température veineuse du patient ce qui nécessite un réglage initial de la valeur de base du paramètre représentatif du stimulateur cardiaque tel que la fréquence de stimulation. Le document WO 85 05279 prévoit un demi-pont de calibrage de la thermistance à une température de référence, mais ne vise pas la résistance de l'ensemble stimulateur-sonde une fois implanté.

On connaît aussi (document FR 1 379 694) un stimulateur cardiaque d'un type différent comportant des moyens d'ajustement de la fréquence au moyen de vis pointeaux modifiant une résistance électrique. Mais la finalité de ce réglage n'est pas destinée à permettre la mise en oeuvre d'un stimulateur piloté par la température veineuse du patient.

L'invention vise à remédier à ces inconvénients et à cet effet elle propose un stimulateur du type mentionné précédemment, qui comporte associée au boitier et interposée en série entre l'un au moins des conducteurs de la sonde et les moyens de stimulation, une résistance électrique réglable depuis l'extérieur du boitier, ayant pour fonction de permettre de régler la résistance électrique du stimulateur avec sa sonde après mise en place quelle que soit la température veineuse et la résistance propre de la sonde.

Selon une autre caractéristique de l'invention, le capteur de température compte un fil de platine logé dans une ampoule étanche en verre et dont les deux parties extrêmes sont émergentes, ladite ampoule étant logée de façon étanche dans un boitier en un matériau inerte au sang et conducteur de l'électricité, l'une des parties exctrêmes du fil de platine étant fixée mécaniquement et en contact électrique avec le boitier, et l'autre partie extrême traversant de façon étanche le boitier.

L'invention présente l'avantage d'une réalisation particulièrement simple, efficace et fiable du capteur de température permettant un montage facile. Du plus, le réglage du "zéro" du stimulateur après implantation et mise en place de la sonde notamment de son extrémité de stimulation est facile puisqu'il suffit de manoeuvrer un organe de réglage mobile associé à la résistance électrique réglable. En particulier cette structure n'est pas consommatrice d'énergie électrique.

Les autres caractéristiques de l'invention résulteront de la description qui suivra en référence aux dessins annexés dans lesquels :

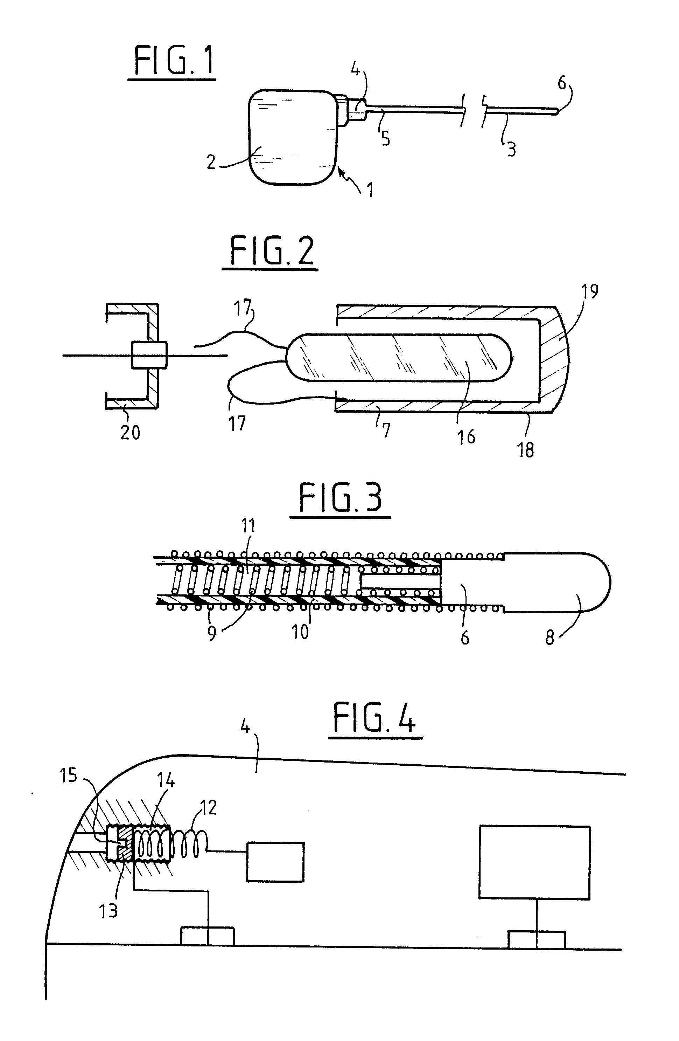

- la figure 1 est une vue générale schématique d'un stimulateur cardiaque selon l'invention.
- la figure 2 est une vue schématique à plus grande échelle du capteur de température de stimulateur cardiaque représenté démonté.
- la figure 3 est une vue schématique partielle représentant l'extrémité de stimulation et de mesure de température.
- la figure 4 est une vue schématique partielle à plus grande échelle du connecteur du stimulateur cardiaque.

L'invention concerne un stimulateur cardiaque 1 du type piloté par la température veineuse du patient appareillé avec ce stimulateur. La stimulateur comporte un boitier 2 et une sonde 3. Le boitier 2 contient, de façon connue en soi, une pile électrique, des moyens de stimulation associés à la pile, non représentés, et un connecteur 4 associé mécaniquement au boitier 2 et électriquement aux moyens de stimulation.

La sonde 3 est destinée à être associée à une première partie extrême 5 au connecteur 4 et comporte à sa second partie extrême 6, d'une part un capteur de température 7, et, d'autre part, au moins un contact de stimulation 8, reliés électriquement au connecteur 4 par des conducteurs 9. De façon également connue en soi, la sonde 3 comporte deux conducteurs 9 spiralés et coaxiaux isolés par une gaine 10 en matériau tel que du silicone, du polyéthilène, du polyuréthane ou tout autre matériau isolant électriquement et biocompatible. Un canal central 11 est ménagé dans la sonde 3 par permettre le logement et la coulissement d'un raidisseur de mise en place de la sonde au moment de l'implantation.

Selon l'invention, le stimulateur 1 comporte, associée au boitier 2 et interposée en série entre l'un au moins des conducteurs 9 de la sonde 3 et les moyens de stimulation une résistance électrique 12 réglable depuis l'extérieur du boitier 2, ayant pour fonction de permettre de régler la résistance électrique du stimulateur 1 avec sa sonde 3 après implantation, quelle que soit la température veineuse et la résistance propre de la sonde.

Par conséquent, le stimulateur cardiaque 1 selon l'invention autorise l'emploi de sondes 3 pouvant présenter des caractéristiques propres notamment de résistances électriques différentes sans pour autant que le réglage nécessaire au bon fonctionnement du stimulateur ne soit rendu complexe ou consommateur en énergie électrique.

La résistance électrique 12 est placée dans le connecteur 4 et comporte un organe de réglage 13, mobile, accessible de l'extérieur par un trou 14 percé dans le connecteur 4 et susceptible d'être obturé, de façon étanche et inviolable par un bouchon rapporté en silicone ou tout autre matériau équivalent (not représenté).

L'organe de réglage mobile 13 est par exemple constitué par une vis montée dans le filtage du trou 14, la vis coopérant électriquement avec la résistance 12 de manière à shunter une partie de cette résistance, de sorte que la résistance effective interposée entre le conducteur 9 correspondant et les moyens de stimulation est réglable, et dépend de la position de l'organe mobile 13. Pour faciliter le régalage, l'organe mobile 13 comporte préférentiellement une tête présentant des reliefs 15 en creux on saillie tels que ceux pouvant coopérer avec un tournevis connu en soi.

Le capteur de température 7 comporte un fil de platine logé dans une ampoule étanche en verre 16 et dont les deux parties extrêmes 17 sont émergentes. L'ampoule de verre 16 est logée de façon étanche dans un boitier 18 en un matériau inerte au sang et conducteur de l'électricité. L'une des parties extrêmes 17 et fixée mécaniquement et en contact électrique avec le boitier 18. L'autre partie extrême 17 traverse de façon étanche le boitier 18.

Le boitier 18 est préférentiellement réalisé en deux parties, à savoir un fond 19 et un couvercle 20 et la partie extrême 17 traversant le boitier est préférentiellement agencée pour traverser le couvercle 20.

Le boitier 18 est préférentiellement réalisé en titane ou en platine, le couvercle 20 étant fixé mécaniquement et de façon étanche au fonds 19 par soudage.

Le capteur de température 7 présente une forme générale cylindrique et se trouve ainsi agencé pour pouvoir être associé commodément notamment par vissage à l'extrémité de la sonde 3.

L'invention concerne également un procédé de réglage d'un stimulateur cardiaque du type décrit précédemment dans lequel on réalise les phases opératoires suivantes :

On implante sur le patient le stimulateur cardiaque (1) c'est-à-dire, le boitier 2 et la sonde 3.

On règle le stimulateur cardiaque 9 sur sa gamme de plus grande sensibilité notamment par télécommande, et ceci de façon connue en soi.

Puis on agit sur la résistance de réglage 12 notamment grâce à l'organe 13 pour ajuster la résistance du stimulateur 1 avec sa sonde 3 donc un paramètre représentatif et significatif du fonctionnement du stimulateur cardiaque 1 measurable depuis l'extérieur, notamment la fréquence de stimulation et ceci pour atteindre la valeur souhaitée.

Lorsque cet ajustement est réalisé, on obture le trou 14. L'organe mobile 13 est agencé de manière à ne se déplacer que lorsqu'il est sollicité par une force extérieure suffisante, de façon positive. Par conséquent, lorsque le réglage est terminé et lorsque le trou 14 est obturé, l'organe de réglage 13 est rendu immobile et il ne peut y avoir déréglage intempestif pour quelle que raison que ce soit. A cet effet, l'organe de réglage 13 lorsqu'il se présente sous la forme d'une vis, peut être monté à frottement dans le trou taraudé 14.

## Revendications

1) Stimulateur cardiaque (1) du type piloté par la température veineuse du patient et comportant un boitier (2) contenant une pile électrique, des moyens de stimulation associés à la pile, un connecteur (4) associé aux moyens de stimulation et une sonde (3) destinée à être associée par une première partie extrême (5) au connecteur (4) et comportant, à sa second partie extrême (6), d'une part un capteur de température (7), et d'autre part au moins un contact de stimulation (8) relié électriquement au connecteur (4) par des conducteurs (9) caractérisé par le fait qu'il comporte, associée au boitier (2) et interposée en série entre l'un au moins des conducteurs (9) de la sonde et les moyens de stimulation, une résistance électrique (12), réglable depuis l'extérieur du boitier (2), ayant pour fonction de permettre de régler la résistance électrique du stimulateur (1) avec sa sonde (3) après mis en place quelle que soit le température veineuse et la résistance propre de la sonde (3).

2) Stimulateur cardiaque selon la revendication 1 caractérisé par le fait que la résistance électrique (12) est placée dans le connecteur

(4) et comporte un organe de réglage (13) mobile, accessible de l'extérieur par un trou (14) percé dans le connecteur (4) et susceptible d'être obturé de façon étanche et inviolable.

3) Stimulateur cardiaque selon l'une quelconque des revendications 1 et 2, caractérisé par le fait que le capteur de température (7) comporte un fil de platine ou similaire logé dans une ampoule étanche en verre (16) et dont les deux parties extrêmes (17) sont émergentes, l'ampoule (16) étant elle-même logée de façon étanche dans un boitier (18) en un matériau inerte au sang et conducteur de l'électricité, l'une des parties extrêmes (17) étant fixée mécaniquement et en contact électrique avec le boitier (18) et l'autre partie extrême (17) traversant de façon étanche le boitier (18).

4) Stimulateur cardiaque selon l'une quelconque des revendications 1, 2 et 3, caractérisé par le fait que le boitier (18) est en deux parties dont un couvercle (20) comporte des moyens de fixation aux conducteurs (9) de la sonde (3).

5) Procédé de réglage d'un stimulateur cardiaque (1) du type piloté par la température veineuse du patient appareillé avec un tel stimulateur, selon l'une quelconque des revendications 1 à 4, caractérisé par le fait que successivement on implante sur le patient le stimulateur cardiaque (1), c'est-à-dire le boitier (2) et la sonde (3), on règle le stimulateur cardiaque (1) sur sa gamme de plus grande sensibilité notamment par télécommande, on agit sur la résistance réglable (12) pour ajuster la résistance du stimulateur cardiaque (1) avec sa sonde (3) donc un paramètre représentatif en significatif, measurable de l'extérieur, notamment la fréquence de stimulation et obtenir la valeur souhaitée ; et, lorsque cet ajustement est terminé, on obture le trou (14) d'accès à la résistance (12).

FIG.1

FIG.2

FIG.3

FIG.4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y,D | WO-A-8 505 279 (ALT) <br> * Résumé; page 4, ligne 14 - page 8, ligne 32 * <br> --- | 1,2,5 | A 61 N 1/365 <br> G 01 K 7/18 |
| Y,D | FR-A-1 379 694 (L'ECECTRONIQUE APPLIQUEE) <br> * En entier * <br> --- | 1,2,5 | |
| A | FR-A-1 342 670 (CIBA) <br> * En entier * <br> --- | 3,4 | |
| A | FR-A- 883 040 (DEUTSCHE GOLD- UND SILBERSCHEIDEANSTALT) <br> * En entier * <br> ----- | 3,4 | |

DOMAINES TECHNIQUES RECHERCHES (Int. Cl.4)

A 61 N
G 01 K

Le présent rapport a été établi pour toutes les revendications

| Lieu de la recherche | Date d'achèvement de la recherche | Examinateur |
|---|---|---|
| LA HAYE | 13-03-1989 | LEMERCIER D.L.L. |

EPO FORM 1503 03.82 (P0402)